# EUROPÄISCHE PATENTSCHRIFT

(11) **EP 4 537 790 B1**
(45) Veröffentlichungstag und Bekanntmachung des Hinweises auf die Patenterteilung: **15.07.2026**
(21) Anmeldenummer: 24202996.5
(22) Anmeldetag: 26.09.2024
(51) Int. Cl.: A61C 19/05, A61B 6/04, A61B 6/51, A61B 5/00

(54) **DETEKTION DER KIEFERBOGENGEOMETRIE**
DETECTION OF JAW ARCH GEOMETRY
DÉTECTION DE LA GÉOMÉTRIE DE L'ARC MAXILLAIRE

(30) Priorität: 09.10.2023 DE 102023127425
(43) Veröffentlichungstag der Anmeldung: 16.04.2025
(73) Patentinhaber: Dürr Dental SE, 74321 Bietigheim-Bissingen (DE)
(72) Erfinder: WENZKO, Robert, 74379 Ingersheim (DE); BEMMANN, Maximilian, 04288 Leipzig (DE)
(74) Vertreter: Frenkel, Matthias Alexander

(56) Entgegenhaltungen:
- DE-A1- 102004 041 440
- DE-A1- 102009 060 390
- KR-A- 20210 055 436
- US-A1- 2008 299 511
- US-A1- 2009 274 267

## Beschreibung

Die vorliegende Erfindung betrifft eine Vorrichtung und ein Verfahren zur Ermittlung von Informationen über zumindest einen Abschnitt eines Zahnbogens.

Bei medizinischen bildgebenden Systemen für die Dentaltechnik ist die Patientenpositionierung von besonderer Bedeutung. Bei heutigen Systemen erfolgt die Patientenpositionierung durch (zahn-)medizinisches Fachpersonal. Die Positionierung ist daher naturgemäß fehleranfällig und fehlerbehaftet. Folglich müssen durch ein medizinisches bildgebendes System durchgeführte Aufnahmen oftmals wiederholt werden oder die Aufnahmen sind von unzureichender Qualität und müssen aufwendig im Nachhinein aufbereitet werden.

Die EP 4 129 191 A1 betrifft ein Verfahren zur Herstellung zur Herstellung digitaler Panoramaaufnahmen mittels extraoraler Röntgengeräte unter Verwendung von Vorwissen über die Anatomie des Patienten, um sich nachgelagerte Software-Lösungen mit aufwändiger Rekonstruktion zu ersparen. Hierbei wird auf vorhandenes Bildmaterial, wie eine oder mehrere vorangegangene Panoramaaufnahmen oder 3D-Aufnahmen oder einen oder mehrere optischer 3D Scans der Kieferbogenform als modellbasiertes Vorwissen über die Anatomie des Patienten zurückgegriffen. Mit anderen Worten wird die Bahnkurve nicht nachberechnet, sondern im Vorfeld über ein Art Künstliche Intelligenz (KI) aus vorhandenem Material vorab berechnet, um eine möglichst genaue Bahnkurve patientenindividuell abzufahren.

Bei der EP 3 688 719 A1 wird ein individuell angepasster Pfad einer Röntgenquelle unter Verwendung einer digitalen 3D Oberflächendarstellung zumindest eines Teils von Zähnen eines Patienten definiert.

Die US 2022/0192616 A1 offenbart eine Reduzierung von Artefakten in der Aufnahme mittels drei Panoramaaufnahmen, wobei die erste Panoramaaufnahme die Basis bildet, die zweite Panoramaaufnahme die Artefakte gewichtet und die Dritte das Resultat der abgezogenen Artefakte aus der zweiten Aufnahme in der ersten Aufnahme darstellt.

Die US 2010/0055634A1 offenbart eine Bissplatte mit einer intraoralen Vibrationsplatte.

Die EP 2 932 903 A1 offenbart ein Kraniostat zur Patientenpositionierung mit einer Stirnhalterung.

Die US 2008/299511 A1 beschreibt ein Verfahren zur Bestimmung der Soll-Relativlage eines Patienten in einem dentalen Panorama-Röntgengerät in dem die Krümmung eines vorderen Bereichs eines Zahnbogens eines Patienten ermittelt wird. Daraus und aus einer Projektionsbogenfläche werden Soll-Lagekoordination für den Patienten berechnet. Zusätzlich wird die Krümmung eines mittleren Bereichs und/oder hinteren Bereichs des Zahnbogens ermittelt und bei der Berechnung der Soll-Lagekoordinaten berücksichtigt.

Die KR 2021 0055436 A beschreibt ein individuelles Zahnpanoramasystem unter Verwendung einer Zahnbogenform und ein Verfahren zu dessen Steuerung.

Die US 2009/274267 A1 beschreibt ein Verfahren zur Durchführung einer panoramischen Röntgenuntersuchung. Das Verfahren umfasst die Erzeugung eines Signals, das einen anatomischen Bereich von Interesse darstellt, beispielsweise über eine Druckkarte, die Berechnung einer Abbildungsschicht auf der Grundlage des erzeugten Signals und die Erzeugung eines Scans auf der Grundlage der berechneten Abbildungsschicht

Ferner bezieht sich die WO 2023/083648 A1 auf ein Verfahren zur Verbesserung eines medizinischen Scans einer Kopfanatomie eines Patienten. Das Verfahren umfasst das Bereitstellen einer Vielzahl von Scan-Datensätzen, die sich auf eine Kopfanatomie beziehen. Das Verfahren umfasst ein Bereitstellen intraoraler Daten, die sich auf ein oder mehrere intraorale Merkmale des Patienten beziehen. Das Verfahren umfasst ein Bestimmen, unter Verwendung der intraoralen Daten, solcher Teile des Scan-Datensatzes, bei deren Erfassung eine Bewegung bezogen auf die intraoralen Merkmale detektiert wurde. Das Verfahren umfasst ein Konstruieren und/oder Korrigieren des medizinischen Scans, von dem Scan-Datensatz, als Antwort auf die Bestimmung.

Selbst bei dem Stand der Technik, der manuelle Eingriffe bei der Patientenpositionierung durch Personal verringert oder sogar auf solche verzichtet, ist der notwendige Aufwand recht hoch, beispielsweise aufgrund von Vorabberechnungen, Nachberechnungen und/oder von zusätzlichen Aufnahmen.

Es besteht daher ein Bedarf nach einer verbesserten Vorrichtung zur Ermittlung von Informationen über zumindest einen Abschnitt eines Zahnbogens sowie nach einem zugehörigen Verfahren.

Die Erfindung ist durch die unabhängigen Ansprüche definiert. Bevorzugte Ausführungsformen sind durch die abhängigen Ansprüche definiert.

Gemäß einem ersten Aspekt der Erfindung wird eine Vorrichtung zur Ermittlung von Informationen über zumindest einen Abschnitt eines Zahnbogens vorgeschlagen. Die Vorrichtung weist eine Aufbissanordnung und eine Auswerteeinheit auf. Die Aufbissanordnung weist mindestens einen Drucksensor auf. Die Aufbissanordnung ist ausgebildet, zwischen zumindest einem Abschnitt einer Zahnreihe eines Oberkiefers und zumindest einem Abschnitt einer Zahnreihe eines Unterkiefers aufgenommen zu werden. Der mindestens eine Drucksensor ist ausgebildet, Druckinformationen über und/oder von zumindest dem Abschnitt der Zahnreihe des Oberkiefers und/oder von zumindest dem Abschnitt der Zahnreihe des Unterkiefers auf den mindestens einen Drucksensor ausgeübten Druck zu erfassen. Die Auswerteeinheit ist ausgebildet, basierend auf den von dem mindestens einen Drucksensor erfassten Druckinformationen, Informationen über zumindest einen Abschnitt eines Zahnbogens zu ermitteln.

Die Ermittlung zumindest des Abschnitts des Zahnbogens mittels Druckinformationen, die durch mindestens einen in einer Aufbissanordnung vorhandenen Drucksensor erfasst werden, erlaubt eine genaue und dennoch einfache Ermittlung.

Als Zahnreihe kann normalerweise die schnurartige Anordnung der Zähne im Ober- und Unterkiefer bezeichnet werden. Die obere und untere Zahnreihe eines erwachsenen Menschen bestehen normalerweise aus jeweils 16 Zähnen. Bei Kindern besteht die obere und die untere Zahnreihe bei vollständiger Ausbildung normalerweise aus jeweils 10 Zähnen.

Der Zahnbogen (auch Alveolarbogen genannt) hat beim Menschen üblicherweise die Form einer Parabel mit dem Scheitelpunkt zwischen den Zähnen Nr. 11 und 21 im Oberkiefer (entsprechend Zähne Nr. 31 und 41 im Unterkiefer). Als ein Zahnbogen kann die parabelförmig / hufeisenförmig verlaufende Zahnreihe des Menschen bezeichnet werden, die im Oberkiefer liegt. Als ein Zahnbogen kann die parabelförmig / hufeisenförmig verlaufende Zahnreihe des Menschen bezeichnet werden, die jeweils im Unterkiefer und im Oberkiefer liegt. Somit kann davon gesprochen werden, dass der Begriff der Zahnreihe sich auf die Aneinanderreihung von Zähnen bezieht, während der Begriff des Zahnbogens, über die bloße Aneinanderreihung von Zähnen hinaus, eine Geometrie und/oder eine Form definiert. Es kann demnach auch davon gesprochen werden, dass die Aufbissanordnung ausgebildet sein kann, zwischen zumindest einem Abschnitt eines Zahnbogens eines Oberkiefers und zumindest einem Abschnitt eines Zahnbogens eines Unterkiefers aufgenommen zu werden. Der Zahnbogen kann auch als Kieferbogen bezeichnet werden.

Die Informationen über zumindest den Abschnitt des Zahnbogens können Informationen über zumindest einen Abschnitt einer Zahnbogenkurve oder einer Zahnbogenform aufweisen. Die Informationen über zumindest einen Abschnitt des Zahnbogens können Informationen über mindestens einen Zahn des Zahnbogens umfassen, beispielsweise Informationen über genau einen Zahn. Es ist auch denkbar, dass die Informationen über zumindest einen Abschnitt des Zahnbogens Informationen über zumindest einen Teil oder Abschnitt eines Zahns des Zahnbogens umfassen.

Die Aufbissanordnung kann eine Aufbissschiene aufweisen oder als Aufbissschiene ausgebildet sein. In einem spezifischen Beispiel kann die Aufbissanordnung eine Aufbissschiene und einen mit der Aufbissschiene direkt oder indirekt verbundenen Schaft aufweisen. Anders ausgedrückt, die Aufbissschiene kann einteilig mit dem Schaft ausgebildet sein, um die Aufbissanordnung zu bilden. Dabei kann die Aufbissschiene unmittelbar in den Schaft übergehen oder es kann mindestens ein weiterer Abschnitt zwischen der Aufbissschiene und dem Schaft vorgesehen sein. Die Aufbissanordnung kann drahtlos und/oder drahtgebunden mit der Auswerteeinheit verbunden oder verbindbar sein, d. h. die Verbindung zwischen der Aufbissanordnung und der Auswerteeinheit kann eine drahtgebundene Verbindung, eine drahtlose Verbindung oder eine Kombination aus beiden aufweisen. Über die drahtlose und/oder drahtgebundene Verbindung kann die Aufbissanordnung die von dem mindestens einen Drucksensor erfassten Druckinformationen an die Auswerteeinheit übermitteln. Die Druckinformationen können Informationen über eine von zumindest dem Abschnitt der Zahnreihe des Oberkiefers und/oder von zumindest dem Abschnitt der Zahnreihe des Unterkiefers auf den mindestens einen Drucksensor ausgeübte oder einwirkende Kraft aufweisen. Die Druckinformationen können demnach auch als Kraftinformationen bezeichnet werden.

Zur Erfassung von Druckinformationen über von zumindest dem Abschnitt der Zahnreihe des Oberkiefers ausgeübten Druck kann der mindestens eine Drucksensor beispielsweise auf einer zu dem Oberkiefer weisenden Seite der Aufbissanordnung angeordnet sein oder über eine dem Oberkiefer weisenden Seite der Aufbissanordnung betätigbar sein. Zur Erfassung von Druckinformationen über von zumindest dem Abschnitt der Zahnreihe des Unterkiefers ausgeübten Druck kann der mindestens eine Drucksensor beispielsweise auf einer zu dem Unterkiefer weisenden Seite der Aufbissanordnung angeordnet sein oder über eine zu dem Unterkiefer weisenden Seite der Aufbissanordnung betätigbar sein. Zur Erfassung von Druckinformationen über von zumindest dem Abschnitt der Zahnreihe des Oberkiefers und von Druckinformationen über von zumindest dem Abschnitt der Zahnreihe des Oberkiefers ausgebübten Druck kann der mindestens eine Drucksensor beispielsweise auf einer zu dem Oberkiefer und auf einer zu dem Unterkiefer weisenden Seite der Aufbissanordnung angeordnet sein oder über eine zu dem Oberkiefer und über eine zu dem Unterkiefer weisenden Seite der Aufbissanordnung betätigbar sein.

Die Auswerteeinheit kann ausgebildet sein, als die Informationen über zumindest den Abschnitt des Zahnbogens, Informationen über zumindest einen Abschnitt eines Zahnbogens des Oberkiefers zu ermitteln. Zusätzlich oder alternativ kann die Auswerteeinheit ausgebildet sein, als die Informationen über zumindest den Abschnitt des Zahnbogens, Informationen über zumindest einen Abschnitt eines Zahnbogens des Unterkiefers zu ermitteln. Anders ausgedrückt, die Auswerteeinheit kann ausgebildet sein, als die Informationen über zumindest den Abschnitt des Zahnbogens, lediglich Informationen über zumindest einen Abschnitt eines Zahnbogens des Oberkiefers zu ermitteln, oder, als die Informationen über zumindest den Abschnitt des Zahnbogens, lediglich Informationen über zumindest einen Abschnitt eines Zahnbogens des Unterkiefers zu ermitteln, oder, als die Informationen über zumindest den Abschnitt des Zahnbogens, sowohl Informationen über zumindest einen Abschnitt eines Zahnbogens des Unterkiefers als auch Informationen über zumindest einen Abschnitt eines Zahnbogens des Oberkiefers zu ermitteln. Die Informationen über zumindest einen Abschnitt eines Zahnbogens des Unterkiefers können Informationen über zumindest einen Abschnitt oder Teil eines Zahns des Unterkiefers oder über mehrere Zähne des Unterkiefers umfassen. Die Informationen über zumindest einen Abschnitt eines Zahnbogens des Oberkiefers können Informationen über zumindest einen Abschnitt oder Teil eines Zahns des Oberkiefers oder über mehrere Zähne des Oberkiefers umfassen.

Die Auswerteeinheit kann ausgebildet sein, als die Informationen über zumindest den Abschnitt des Zahnbogens, Informationen über den gesamten Zahnbogen des Oberkiefers zu ermitteln. Zusätzlich oder alternativ kann die Auswerteeinheit ausgebildet sein, als die Informationen über zumindest den Abschnitt des Zahnbogens, Informationen über den gesamten Zahnbogens des Unterkiefers zu ermitteln.

Die Auswerteeinheit kann ausgebildet sein, als die Informationen über zumindest den Abschnitt des Zahnbogens, Informationen über zumindest einen Abschnitt eines, aus dem Zahnbogen des Oberkiefers und dem Zahnbogen des Unterkiefers, gemittelten Zahnbogens zu ermitteln. Der gemittelte Zahnbogen kann auf verschiedene Weisen aus dem Zahnbogen des Oberkiefers und dem Zahnbogen des Unterkiefers ermittelt werden. Beispielsweise kann in einer einfachen Ausgestaltung schlicht der Mittelwert aus den Informationen über zumindest den Abschnitt des Zahnbogens des Oberkiefers und den Informationen über zumindest den Abschnitt des Zahnbogens des Unterkiefers gebildet werden, um die Informationen über zumindest den Abschnitt des gemittelten Zahnbogens zu ermitteln. Alternativ können die Informationen über zumindest den Abschnitt des Zahnbogens des Oberkiefers und die Informationen über zumindest den Abschnitt des Zahnbogen des Unterkiefers gemäß einer vorbestimmten Gewichtung oder einem vorbestimmten Gewichtungsschema miteinander kombiniert werden, um die Informationen über zumindest den Abschnitt des gemittelten Zahnbogens zu ermitteln. Die Auswerteeinheit kann ausgebildet sein, als die Informationen über zumindest den Abschnitt des Zahnbogens, Informationen über den gesamten gemittelten Zahnbogen zu ermitteln.

Die Auswerteeinheit kann ausgebildet sein, basierend auf den von dem mindestens einen Drucksensor erfassten Druckinformationen, Informationen über Geometrie und/oder eine Breite und/oder einer Lage eines Zahnbogens, beispielsweise eines Zahnbogens des Oberkiefers und/oder eines Zahnbogens des Unterkiefers, zu ermitteln.

Die Auswerteeinheit kann ausgebildet sein, basierend auf den von dem mindestens einen Drucksensor erfassten Druckinformationen, Informationen über eine Breite und/oder einer Lage eines den Oberkiefer und den Unterkiefer aufweisenden Kiefers zu ermitteln. Zusätzlich oder alternativ kann die Auswerteeinheit ausgebildet sein, basierend auf den von dem mindestens einen Drucksensor erfassten Druckinformationen, Informationen über eine Breite und/oder einer Lage des Oberkiefers zu ermitteln. Zusätzlich oder alternativ kann die Auswerteeinheit ausgebildet sein, basierend auf den von dem mindestens einen Drucksensor erfassten Druckinformationen, Informationen über eine Breite und/oder einer Lage des Unterkiefers zu ermitteln.

Der mindestens eine Drucksensor kann ausgebildet und angeordnet sein, als die Druckinformationen, Informationen über einen oder mehrere Aufbisspunkte in der Zahnreihe des Oberkiefers und/oder in der Zahnreihe des Unterkiefers zu erfassen. Der mindestens eine Drucksensor kann ausgebildet und angeordnet sein, als die Druckinformationen, lediglich Informationen über einen oder mehrere Aufbisspunkte in der Zahnreihe des Oberkiefers zu erfassen. Hierfür kann der mindestens eine Drucksensor auf einer zu dem Oberkiefer weisenden Seite der Aufbissanordnung angeordnet sein oder über eine zu dem Oberkiefer weisenden Seite der Aufbissanordnung betätigbar sein. Der mindestens eine Drucksensor kann ausgebildet und angeordnet sein, als die Druckinformationen, lediglich Informationen über einen oder mehrere Aufbisspunkte in der Zahnreihe des Unterkiefers zu erfassen. Hierfür kann der mindestens eine Drucksensor auf einer zu dem Unterkiefer weisenden Seite der Aufbissanordnung angeordnet sein oder über eine zu dem Unterkiefer weisenden Seite der Aufbissanordnung betätigbar sein. Der mindestens eine Drucksensor kann ausgebildet und angeordnet sein, als die Druckinformationen, sowohl Informationen über einen oder mehrere Aufbisspunkte in der Zahnreihe des Oberkiefers als auch Informationen über einen oder mehrere Aufbisspunkte in der Zahnreihe des Unterkiefers zu erfassen. Hierfür kann der mindestens eine Drucksensor auf einer zu dem Oberkiefer weisenden Seite der Aufbissanordnung und auf einer zu dem Unterkiefer weisenden Seite der Aufbissanordnung angeordnet sein. Zusätzlich oder alternativ kann der mindestens eine Drucksensor über eine zu dem Oberkiefer weisenden Seite der Aufbissanordnung und über eine zu dem Unterkiefer weisenden Seite der Aufbissanordnung betätigbar sein.

Die Ermittlung von Aufbisspunkten mittels des mindestens einen Drucksensors in der Aufbissanordnung erlaubt eine besonderes genaue und dennoch einfache Ermittlung der Informationen über zumindest den Abschnitt des Zahnbogens.

Der mindestens eine Drucksensor kann ausgebildet und angeordnet sein, als die Druckinformationen, Informationen über bukkale und/oder palatinale Positionen von einem oder mehreren Zahnhöckern und/oder von einem oder mehreren Schneideflächen von Zähnen der Zahnreihe des Oberkiefers und/oder von Zähnen der Zahnreihe des Unterkiefers zu erfassen. Dabei ist bukkal (oder buccal) in der Zahnmedizin normalerweise der Fachausdruck für den Wangen zugewandte Zahnoberflächen, d. h. für Außenseiten der Zähne. Ferner ist palatinal in der Zahnmedizin normalerweise der Fachausdruck für dem Gaumen zugewandte Zahnoberflächen, d. h. für Innenseiten der Zähne. Zusätzlich oder alternativ kann der mindestens eine Drucksensor ausgebildet und angeordnet sein, als die Druckinformationen, Informationen über eine oder mehrere Zahnfehlstellungen und/oder einen schrägen Biss von einem oder mehreren Zähnen der Zahnreihe des Oberkiefers und/oder von einem oder mehreren Zähnen der Zahnreihe des Unterkiefers zu erfassen. Die eine oder mehreren Zahnfehlstellungen können beispielsweise direkt aus den Druckinformationen, beispielsweise einem Druckaufbiss, ableitbar sein.

Der mindestens eine Drucksensor kann ein Sensor-Array oder Sensorfeld aufweisen, insbesondere ein Sensor-Array oder Sensorfeld mit Einzelsensoren und/oder Dünnfilmsensoren und/oder Foliensensoren. In einer spezifischen Ausgestaltung kann der mindestens eine Drucksensor als Sensor-Array oder Sensorfeld ausgebildet sein, insbesondere als ein Sensor-Array oder Sensorfeld mit Einzelsensoren und/oder Dünnfilmsensoren und/oder Foliensensoren.

Die Auswerteeinheit kann ausgebildet sein, basierend auf den Informationen über zumindest den Abschnitt des Zahnbogens, Informationen über eine Bahnkurve eines medizinischen bildgebenden Geräts, insbesondere eines extraoralen Röntgengeräts, zu ermitteln. Das extraorale Röntgengerät kann als ein Panorama-Röntgengerät ausgebildet sein. Das Röntgengerät kann einen Röntgenstrahler und einen Röntgendetektor aufweisen. Der Röntgenstrahler und/oder der Röntgendetektor kann/können sich auf einer zu bestimmenden Bahnkurve bewegen, insbesondere um einen Teil, beispielsweise Kopf, eines Patienten bewegen. In dem medizinischen bildgebenden Gerät kann eine Standard-Bahnkurve hinterlegt oder abgespeichert sein, die unabhängig von patientenspezifischen Informationen abgefahren wird. Basierend auf den Informationen über zumindest den Abschnitt des Zahnbogens kann eine patientenspezifische Bahnkurve des medizinischen bildgebenden Geräts ermittelt werden. Beispielsweise kann die Standard-Bahnkurve basierend auf den Informationen über zumindest den Abschnitt des Zahnbogens angepasst werden, um die patientenspezifische Bahnkurve zu ermitteln. Die Anpassung kann erfolgen unter Berücksichtigung von Abweichungen zwischen einem Standard-Zahnbogen und den Informationen über zumindest den Abschnitt des Zahnbogens. Der Standard-Zahnbogen kann in dem medizinischen bildgebenden Gerät hinterlegt oder gespeichert sein. Allgemein kann die Auswerteeinheit ausgebildet sein, basierend auf den Informationen über zumindest den Abschnitt des Zahnbogens, eine patientenspezifische Bahnkurve eines medizinischen bildgebenden Geräts vollständig zu ermitteln. Alternativ kann die Auswerteeinheit ausgebildet sein, basierend auf den Informationen über zumindest den Abschnitt des Zahnbogens, einen oder mehrere Parameter einer vorhandenen Bahnkurve anzupassen, beispielsweise eine Kontur und/oder eine Position der Bahnkurve im Raum anzupassen, um die patientenspezifische Bahnkurve des medizinischen bildgebenden Geräts zu ermitteln.

Die Ermittlung der Informationen über eine Bahnkurve eines medizinischen bildgebenden Geräts basierend auf den Informationen über zumindest den Abschnitt des Zahnbogens erlaubt eine genaue und patientenspezifische Ermittlung der Informationen über die Bahnkurve. Insbesondere kann eine Ermittlung der Informationen über die Bahnkurve ohne oder zumindest nahezu ohne menschliche Interaktion bei der Positionierung eines Patienten ermöglicht werden.

Die Auswerteeinheit kann ausgebildet sein, basierend auf den von dem mindestens einen Drucksensor erfassten Druckinformationen, Informationen über eine Sagittalebene zu ermitteln. Die Sagittalebene ist eine Ebene, die normalerweise vertikal parallel zur Sutura sagittalis (Pfeilnaht des Schädels) verläuft.

Die Auswerteeinheit kann ferner ausgebildet sein, basierend auf den ermittelten Informationen über die Sagittalebene, Informationen über eine/die Bahnkurve eines medizinischen bildgebenden Geräts anzupassen. Dadurch kann die patientenspezifische Bahnkurve noch besser und/oder genauer patientenspezifisch angepasst werden.

Die Aufbissanordnung weist ferner mindestens eine Messeinrichtung auf. Die Messeinrichtung weist mindestens einen Dehnungsmessstreifen an einer Oberseite eines Schaftes der Aufbissanordnung und/oder mindestens einen Dehnungsmessstreifen an einer Unterseite des Schaftes der Aufbissanordnung auf. Anders ausgedrückt, kann die Messeinrichtung lediglich mindestens einen Dehnungsmessstreifen an einer Oberseite eines Schaftes der Aufbissanordnung aufweisen. Alternativ kann die Messeinrichtung lediglich mindestens einen Dehnungsmessstreifen an einer Unterseite des Schaftes der Aufbissanordnung aufweisen. Alternativ kann die Messeinrichtung sowohl mindestens einen Dehnungsmessstreifen an einer Oberseite eines Schaftes der Aufbissanordnung als auch mindestens einen Dehnungsmessstreifen an einer Unterseite des Schaftes der Aufbissanordnung aufweisen. In einem spezifischen Beispiel kann die Messeinrichtung als mindestens ein Dehnungsmessstreifen an einer Oberseite eines Schaftes der Aufbissanordnung und/oder als mindestens ein Dehnungsmessstreifen an einer Unterseite des Schaftes der Aufbissanordnung ausgebildet sein. Durch die Anordnung von Dehnungsmesstreifen an der Oberseite und/oder der Unterseite des Schaftes kann sich eine wellenartige Form (Wellenform) des Schaftes ergeben. Unter Dehnungsmessstreifen (DMS) werden normalerweise Messeinrichtungen zur Erfassung von dehnenden und/oder stauchenden Verformungen verstanden. DMS ändern schon bei geringen Verformungen ihren elektrischen Widerstand und können als Dehnungssensoren eingesetzt werden. Aufgebracht auf die Aufbissanordnung können sie erfassen, wenn sich die Aufbissanordnung minimal verformt. Diese Verformung (Dehnung) führt dann zur Veränderung des Widerstands des DMS. Die Veränderung des Widerstands des DMS kann von der Auswerteeinheit ausgewertet werden. Die Aufbissanordnung kann zumindest abschnittsweise schräg im Raum verlaufend angeordnet sein. Beispielsweise kann zumindest ein Abschnitt der Aufbissanordnung gegenüber einer Okklusalebene (Okklusionsebene) und/oder gegenüber einer Horizontalen geneigt sein oder schräg verlaufen.

Die Auswerteeinheit kann ausgebildet sein, basierend auf von der mindestens einen Messeinrichtung erfassten Informationen, Informationen über eine Neigung einer Okklusalebene (auch als Okklusionsebene bezeichnet) zu ermitteln. Falls auf Dehnungsmessstreifen an der Ober- und Unterseite des Schaftes der Aufbissanordnung beispielsweise gleich große Werte ausgeben / liefern, wird keine Kraft auf den Schaft durch den Patienten ausgeübt. Somit ist auch die Okklusalebene in der richtigen Lage. Anders ausgedrückt, wird keine Kraft auf den Schaft durch den Patienten ausgeübt, bildet die Aufbissanordnung inklusive der Aufbissschiene und dem Schaft die perfekte Position, z. B. Neigung und Drehung, der Okklusalebene ab. Wird eine ungleichmäßige Kraftverteilung zwischen Ober- und Unterseite des Schaftes erkannt, kann die Lage der Okklusalebene entsprechend angepasst werden. Als Okklusionsebene / Okklusalebene wird normalerweise die Ebene bezeichnet, auf der die Zähne des Oberkiefers und des Unterkiefers aufeinanderstoßen. Die ermittelten Informationen über die Okklusalebene können von der Auswerteeinheit verwendet werden, um die Informationen über zumindest den Abschnitt des Zahnbogens anzupassen. Die ermittelten Informationen über die Okklusalebene können von der Auswerteeinheit verwendet werden, um die Informationen über die Bahnkurve des medizinischen bildgebenden Geräts anzupassen.

Gemäß einem zweiten Aspekt wird ein Verfahren zur Ermittlung von Informationen über zumindest einen Abschnitt eines Zahnbogens vorgeschlagen. Das Verfahren umfasst ein Bereitstellen einer Aufbissanordnung mit mindestens einem Drucksensor und mindestens einer Messeinrichtung oder mindestens einem Dehnungsmessstreifen an einer Oberseite eines Schaftes der Aufbissanordnung und/oder mindestens einem Dehnungsmessstreifen an einer Unterseite des Schaftes der Aufbissanordnung. Das Verfahren umfasst ferner ein Aufnehmen der Aufbissanordnung zwischen zumindest einem Abschnitt einer Zahnreihe eines Oberkiefers und zumindest einem Abschnitt einer Zahnreihe eines Unterkiefers. Das Verfahren umfasst ferner ein Erfassen von Druckinformationen über von zumindest dem Abschnitt der Zahnreihe des Oberkiefers und/oder von zumindest dem Abschnitt der Zahnreihe des Unterkiefers auf den mindestens einen Drucksensor der Aufbissanordnung ausgeübten Druck. Das Verfahren umfasst ferner ein Ermitteln, durch eine Auswerteeinheit, basierend auf den von dem mindestens einen Drucksensor erfassten Druckinformationen, von Informationen über zumindest einen Abschnitt eines Zahnbogens.

Ein dritter Aspekt bezieht sich auf ein Computerprogramm mit Programmcodemitteln, das, wenn es in einen Computer oder einen Prozessor (beispielsweise einen Mikroprozessor, Mikrocontroller oder digitalen Signalprozessor (DSP)) geladen ist, oder auf einem Computer oder Prozessor (z.B. einem Mikroprozessor, Mikrocontroller oder DSP) läuft, den Computer oder Prozessor (z.B. Mikroprozessor, Mikrocontroller oder DSP) dazu veranlasst, einen oder mehrere Schritte oder alle Schritte der zuvor in Bezug auf die Auswerteeinheit beschriebenen Verfahrensschritte auszuführen. Zudem wird ein Programmspeichermedium oder Computerprogrammprodukt mit dem genannten Computerprogramm bereitgestellt.

Ferner kann beispielsweise das Computerprogramm gemäß dem dritten Aspekt in der Auswerteeinheit der Vorrichtung gemäß dem ersten Aspekt gespeichert sein und die Auswerteeinheit dazu veranlassen, einen oder mehrere oder alle der zuvor in Bezug auf die Auswerteeinheit beschriebenen Aspekte und/oder Schritte des Verfahrens gemäß dem zweiten Aspekt auszuführen. Ferner kann beispielsweise das Computerprogramm gemäß dem dritten Aspekt in der Auswerteeinheit der Vorrichtung gemäß dem ersten Aspekt gespeichert sein und die Auswerteeinheit dazu veranlassen, einen oder mehrere oder alle der zuvor in Bezug auf die Auswerteeinheit beschriebenen Aspekte und/oder Merkmale auszuführen.

Ein vierter Aspekt bezieht sich auf ein medizinisches bildgebendes Gerät, beispielsweise ein Röntgengerät, insbesondere ein Panorama-Röntgengerät, mit der hierin beschriebenen Vorrichtung gemäß dem ersten Aspekt und/oder mit der in Bezug auf die Vorrichtung gemäß dem ersten Aspekt beschriebenen Auswerteeinheit.

Auch wenn einige der voranstehend beschriebenen Aspekte in Bezug auf die Auswerteeinheit beschrieben wurden, so können diese Aspekte auch in entsprechender Weise in dem medizinischen bildgebenden Gerät, dem Verfahren oder einem das Verfahren oder die Recheneinheit implementierenden Computerprogramm realisiert werden. Genauso können die voranstehend in Bezug auf die Verfahren beschriebenen Aspekte in dem medizinischen bildgebenden Gerät, dem Verfahren oder einem das Verfahren oder die Recheneinheit implementierenden Computerprogramm realisiert werden.

Die vorliegende Offenbarung soll weiter anhand von Figuren erläutert werden. Diese Figuren zeigen schematisch:
- Figur 1: einen Schädel mit einer Aufbissschiene;
- Figur 2: eine Vorrichtung gemäß einem Ausführungsbeispiel mit einer Aufbissanordnung und einer Auswerteeinheit;
- Figur 3: ein Flussdiagramm eines Verfahrens gemäß einem Ausführungsbeispiel;
- Figur 4: durch die Vorrichtung aus Figur 2 beispielhaft ermittelte Aufbisspunkte einer Zahnreihe;
- Figur 5: eine durch die Vorrichtung aus Figur 2 beispielhaft ermittelte Zahnbogenkurve;
- Figur 6: mögliche Anpassungen des Zahnbogens aus Figur 5 gemäß einer Breite des Kiefers;
- Figur 7: eine beispielhafte Rotationsanpassung der in Figur 6 beispielhaft ermittelten Zahnbogenkurve; und
- Figur 8: eine Variante der Vorrichtung aus Figur 2 mit Dehnungsmessstreifen.

Im Folgenden werden, ohne hierauf beschränkt zu sein, spezifische Details dargelegt, um ein vollständiges Verständnis der vorliegenden Offenbarung zu liefern. Es ist einem Fachmann jedoch klar, dass die vorliegende Offenbarung in anderen Ausführungsbeispielen verwendet werden kann, die von den nachfolgend dargelegten Details abweichen können. Beispielsweise werden im Folgenden spezifische Konfigurationen und Ausgestaltungen einer Auswerteeinheit und/oder einer Aufbissanordnung beschrieben, die nicht als einschränkend anzusehen sind.

Es ist dem Fachmann klar, dass die nachfolgend dargelegten Erklärungen unter Verwendung von Hardwareschaltungen, Softwaremitteln oder einer Kombination davon implementiert sein/werden können. Die Softwaremittel können im Zusammenhang stehen mit programmierten Mikroprozessoren, einer künstlichen Intelligenz oder einem allgemeinen Computer, einer ASIC (Application Specific Integrated Circuit; auf Deutsch: anwendungsspezifische integrierte Schaltung) und/oder DSPs (Digital Signal Processors; auf Deutsch: digitale Signalprozessoren). Es ist zudem klar, dass auch dann, wenn die nachfolgenden Details in Bezug auf ein Verfahren beschrieben werden, diese Details auch in einer geeigneten Vorrichtungseinheit, einem Computerprozessor oder einem mit einem Prozessor verbundenen Speicher realisiert sein können, wobei der Speicher mit einem oder mehreren Programmen versehen ist, die das Verfahren durchführen, wenn sie durch den Prozessor ausgeführt werden.

Die Ausführungsbeispiele können realisiert sein und/oder verwendet werden in verschiedenen medizinischen bildgebenden Geräten. Rein beispielhaft seien hier Röntgengeräte, insbesondere Panorama-Röntgengeräte, genannt.

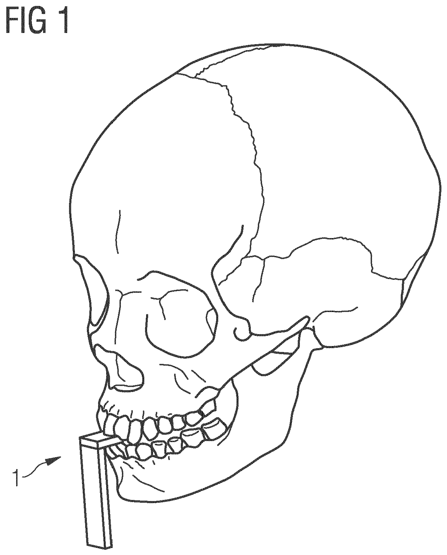

Figur 1 zeigt schematisch einen Schädel eines Patienten sowie eine herkömmliche Aufbissschiene 1. Wie zu erkennen, beißt der Patient vor und/oder während der Durchführung einer Röntgenaufnahme(n) in die Aufbissschiene 1. Über den Eingriff der Zähne in die Aufbissschiene erhofft man sich eine teilweise Fixierung des Kopfes und insbesondere, dass der Kopf des Patienten während der Röntgenaufnahme(n) möglichst ruhig und in einer festen, beispielsweise kalibrierten, Position verbleibt. Insbesondere wird durch den Aufbiss versucht, eine Translation des Schädels zu begrenzen oder zumindest nahezu zu verhindern. Auf diese Weise erhofft man sich, möglichst genaue und scharfe Röntgenaufnahmen erstellen zu können.

In der Praxis zeigt sich jedoch, dass diese Vorgehensweise fehleranfällig ist. Zum einen verbleibt der Kopf für gewöhnlich nicht in der erhofften Ruhestellung. Zum anderen beruht die Positionierung auf Erfahrungswerten des Praxispersonals und unterliegt dadurch menschlichen Fehlern und Fehleinschätzungen.

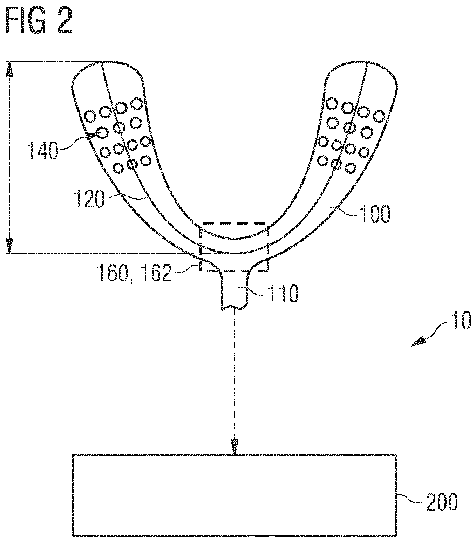

In Figur 2 ist eine Vorrichtung 10 gemäß einem Ausführungsbeispiel schematisch dargestellt. Die Vorrichtung 10 dient zur Ermittlung von Informationen über zumindest einen Abschnitt eines Zahnbogens. Die Vorrichtung 10 weist eine Aufbissanordnung 100 und eine Auswerteeinheit 200 auf. Die Aufbissanordnung 100 weist mindestens einen Drucksensor 140 auf. In der beispielhaften Ausgestaltung gemäß Figur 2 ist der mindestens eine Drucksensor 140 beispielhaft als eine Vielzahl von Drucksensoren 140 ausgebildet. Anders ausgedrückt, die Aufbissanordnung 100 weist beispielhaft eine Vielzahl von Drucksensoren 140 auf. Die beispielhafte Ausgestaltung der Aufbissanordnung 100 aus Figur 2 weist eine Aufbissschiene 120 sowie einen Schaft 110 auf. Die Aufbissschiene 120 ist beispielsweise der Teil der Aufbissanordnung 100, der zwischen Zähnen eines Patienten aufgenommen oder platziert wird. In der beispielhaft in Figur 2 gezeigten Ausgestaltung sind mehrere Drucksensoren 140 der Vielzahl von Drucksensoren auf einer Seite / an einem Ende (in Figur 2 links) und mehrere Drucksensoren 140 der Vielzahl von Drucksensoren auf einer anderen Seite / an einem anderen Ende (in Figur 2 rechts) angeordnet. Die Anzahl an Drucksensoren auf der einen Seite entspricht beispielhaft, und ohne hierauf beschränkt zu sein, in Figur 2 der Anzahl an Drucksensoren auf der anderen Seite. Die Drucksensoren können über die Aufbissschiene 120 verteilt angeordnet sein. Sie können von einer Seite (Oberseite oder Unterseite) oder von beiden Seiten (Oberseite und Unterseite) der Aufbissschiene 120 aus auslösbar sein.

Die Aufbissanordnung 100, insbesondere die Aufbissschiene 120 in Figur 2, ist ausgebildet, zwischen zumindest einem Abschnitt einer Zahnreihe eines Oberkiefers und zumindest einem Abschnitt einer Zahnreihe eines Unterkiefers aufgenommen zu werden. Die Vielzahl an Drucksensoren 140 aus Figur 2, als Beispiel für mindestens einen Drucksensor 140, ist ausgebildet, Druckinformationen über von zumindest dem Abschnitt der Zahnreihe des Oberkiefers und/oder von zumindest dem Abschnitt der Zahnreihe des Unterkiefers auf die Vielzahl von Drucksensoren 140 ausgeübten Druck zu erfassen. Die Auswerteeinheit 200 ist ausgebildet, basierend auf zumindest einer Teilmenge der von der Vielzahl von Drucksensoren 140 erfassten Druckinformationen, Informationen über zumindest einen Abschnitt eines Zahnbogens zu ermitteln. Zur Übermittlung der von den Drucksensoren 140 erfassten Druckinformationen an die Auswerteeinheit können die Drucksensoren 140 drahtlos und/oder drahtgebunden mit der Auswerteeinheit 200 verbunden sein.

Die Aufbissschiene 120 kann eine Zahnbogenform oder eine Teil-Zahnbogenform aufweisen. Das Aufbissschiene 120 kann mindestens ein Sensorarray als Drucksensoren 140 aufweisen. Beispielsweise kann die Aufbissschiene 120 ein erstes Sensorarray auf der einen Seite (Oberseite) und ein zweites Sensorarray auf der anderen Seite (Unterseite) aufweisen. Das mindestens eine Sensorarray kann eine Fläche zur Vermessung der Kiefergeometrie bilden. Beispielsweise kann ein erstes Sensorarray eine Fläche zur Vermessung der Oberkiefergeometrie an/auf der Aufbissschiene 120 bilden und es kann ein zweites Sensorarray eine Fläche zur Vermessung der Unterkiefergeometrie an/auf der Aufbissschiene 120 bilden. Dazu können Einzelsensoren- oder Foliensensoren genutzt werden. Anschließend kann von der Auswerteeinheit 200 aus diesen Daten ein, beispielsweise gemittelter, patientenspezifischer Kieferbogen bestimmt werden. Unter Verwendung des Kieferbogens kann von der Auswerteeinheit 200 eine zumindest (nahezu) perfekt passende, patientenspezifische Geräte-Bewegungskurve bestimmt / berechnet / gewählt werden. Des Weiteren kann eine Schichtlage (ein scharfer Bereich) beispielsweise bei einer Panoramabild-Bild- oder auch bei einer 3D-Modell-Erstellung patientenspezifisch angepasst werden. Somit wird die Qualität der Bildrohdaten so hochwertig, dass eine Bildnachbearbeitung entfallen oder auf ein Minimum reduziert werden kann.

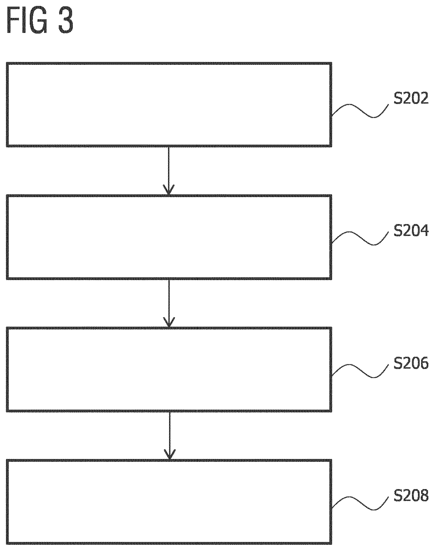

Figur 3 zeigt ein Flussdiagramm eines Verfahrens zur Ermittlung von Informationen über zumindest einen Abschnitt eines Zahnbogens, das in der oder durch die Vorrichtung 100 aus Figur 1 ausgeführt werden kann. In Schritt S202 wird eine Aufbissanordnung 100 mit mindestens einem Drucksensor 140, und bezugnehmend auf die Ausgestaltung aus Figur 2 mit einer Vielzahl von Drucksensoren 140, bereitgestellt. In Schritt S204 wird die Aufbissanordnung 100, und bezugnehmend auf die Ausgestaltung aus Figur 2 zumindest ein Abschnitt der Aufbissschiene 120, zwischen zumindest einem Abschnitt einer Zahnreihe eines Oberkiefers und zumindest einem Abschnitt einer Zahnreihe eines Unterkiefers aufgenommen oder platziert. In Schritt S206 werden Druckinformationen über von zumindest dem Abschnitt der Zahnreihe des Oberkiefers und/oder von zumindest dem Abschnitt der Zahnreihe des Unterkiefers auf die Vielzahl von Drucksensoren 140 der Aufbissanordnung 100, und bezugnehmend auf Figur 2 genauer gesagt der Aufbissschiene 120, ausgeübten Druck erfasst werden. In Schritt S208 werden, durch die Auswerteeinheit 200, basierend auf den von der Vielzahl von Drucksensoren 140 erfassten Druckinformationen, Informationen über zumindest einen Abschnitt eines Zahnbogens ermittelt.

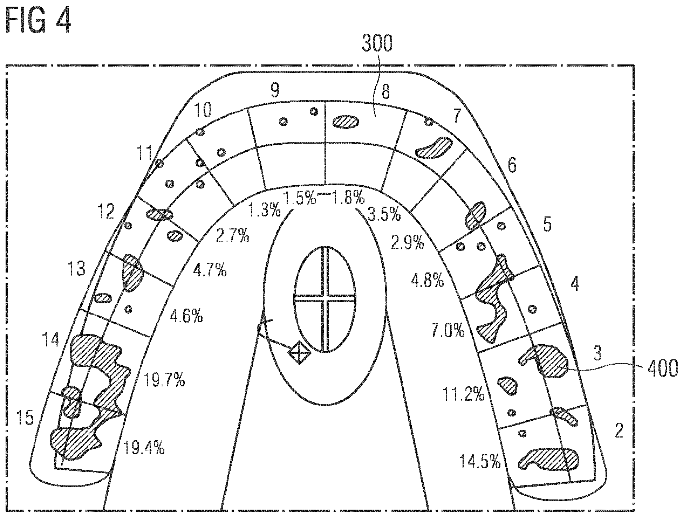

Weitere Details der Vorrichtung 100 aus Figur 2 und des Verfahrens aus Figur 3 werden nun in Bezug auf die Figuren 4 bis 8 beschrieben. Auch wenn in den Figuren 4 bis 8 beispielhaft teilweise nur auf den Oberkiefer Bezug genommen wird, gelten die Ausführungen entsprechend auch für den Unterkiefer.

In Figur 4 werden Aufbisspunkte / Aufbissbereiche 400 einer Zahnreihe 300 des Oberkiefers dargestellt. Im Folgenden wird der Einfachheit halber stets von Aufbisspunkten 400 gesprochen, auch wenn es sich teilweise um Bereiche handelt und dies somit auch als Aufbissbereiche 400 bezeichnet werden könnten. Die Drucksensoren 140 erfassen von der Zahnreihe 300 des Oberkiefers auf die Drucksensoren 140 der Aufbissschiene 120 ausgeübten Druck. Die entsprechenden Druckinformationen werden an die Auswerteeinheit 200 übermittelt, beispielsweise von der Auswerteeinheit 200 abgefragt oder direkt nach Erfassung der Drucksensoren 140 an die Auswerteeinheit 200 übermittelt. Die von der Auswerteeinheit 200 erhaltenen Druckinformationen können als Aufbisspunkte 400 der Zahnreihe 300 des Oberkiefers, wie in Figur 4 beispielhaft gezeigt, veranschaulicht werden.

Genauer gesagt werden mittels der Drucksensoren 140 die Aufbisspunkte 400 der jeweiligen Einzelzähne der Zahnreihe 300 des Oberkiefers und der Zahnreihe des Unterkiefers erfasst/gemessen. Aus den Aufbisspunkten 400 kann die Auswerteeinheit 200 beispielsweise bukkale und/oder palatinale Positionen einzelner Zähne, insbesondere einzelner Zahnhöcker und/oder Schneideflächen, bestimmen. Zusätzlich oder alternativ kann die Auswerteeinheit 200 aus den Aufbisspunkten 400 beispielsweise eine oder mehrere Zahnfehlstellungen und/oder einen schrägen Biss ermitteln. Die eine oder die mehreren Zahnfehlstellungen kann die Auswerteeinheit 200 beispielsweise unmittelbar aus den Aufbisspunkten 400 ableiten.

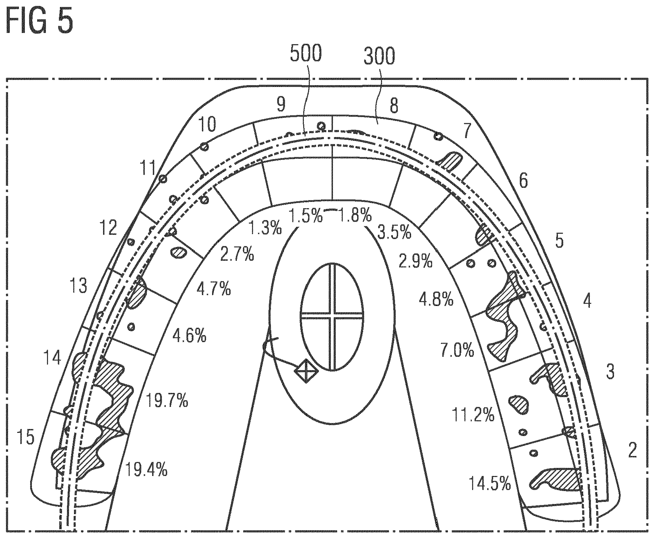

Die Auswerteeinheit 200 wertet die Aufbisspunkte 400 aus und erstellt aus den Aufbisspunkten 400 Informationen über einen patientenspezifischen Zahnbogen, die in Figur 5 als Zahnbogenkurve 500 entlang der Zahnreihe 300 veranschaulicht sind. Diese Zahnbogenkurve 500 ist beispielhaft in Figur 5 veranschaulicht und kann von der beispielhaft gezeigten Form abweichen. Der Zahnbogen (auch Alveolarbogen genannt) hat, wie beim Menschen üblich, die Form einer Parabel mit dem Scheitelpunkt zwischen den Zähnen Nr. 11 und 21 im Oberkiefer (entsprechend Zähne Nr. 31 und 41 im Unterkiefer). Der Oberkieferzahnbogen ist im Normalfall etwas größer als der des Unterkiefers. Die Zahnbogenkurve 500 wird von der Auswerteeinheit 200 unter Berücksichtigung der Aufbisspunkte 400 ermittelt.

Gemäß der obigen Beschreibung in Bezug auf den Oberkiefer kann auf entsprechende Weise auch die Zahnbogenkurve des Unterkiefers von der Auswerteeinheit 200 ermittelt werden. Durch ein Zusammenführen der Daten von Ober- und Unterkiefer kann eine möglichst exakte parabelförmige, patientenspezifische Zahnkurve / Zahnbogenkurve ermittelt werden.

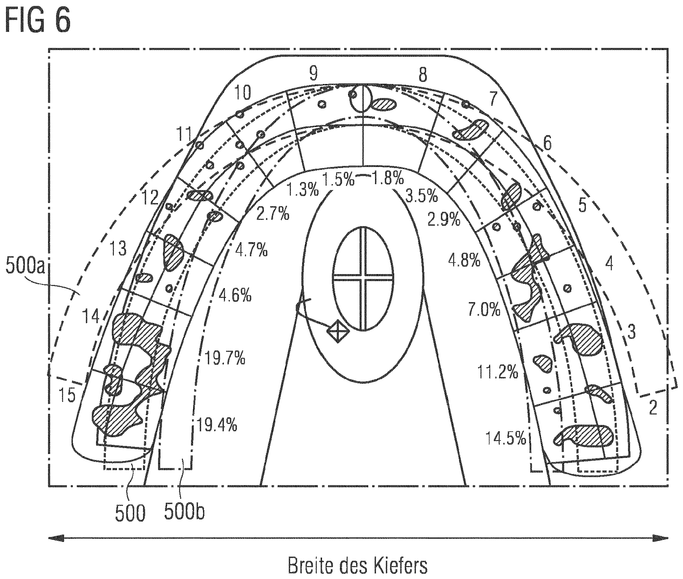

In Figur 6 wird schematisch gezeigt, wie die Auswerteeinheit 200 die ermittelte Zahnbogenkurve 500 anpassen kann. Die ermittelte Zahnbogenkurve kann zunächst durch eine sogenannte "Region of Interest (ROI)" auf Deutsch: relevanter Messbereich), also einen interessierenden Bereich, ergänzt werden. Die ROI kann um die Zahnbogenkurve 500 liegen. Die ROI ist durch den breiteren Bereich um die Zahnbogenkurve 500 in Figur 6 veranschaulicht. Beispielsweise kann die Zahnbogenkurve in der Mitte der ROI liegen. Die Stärke der ROI steigt im Bereich des Kieferbogens von vorne (Frontzähne) nach hinten (Backenzähne) an und ist daher im Bereich des Kieferbogens unterschiedlich (Frontzähne = dünne ROI -> Backenzähne = dicke ROI), wie dies in Figur 6 veranschaulicht ist. Zur Erzielung einer bestmöglichen Bildqualität sollte die ROI immer nur so dick oder breit gewählt sein wie nötig und daher nicht unnötig groß und ungenau sein. In der Figur 6 und den folgenden Figuren ist die ROI bereits bei der Darstellung der Zahnbogenkurve 500 des Oberkiefers zu sehen. Dies ist nicht als einschränkend zu verstehen, sondern dient lediglich der Illustration. Das heißt, es kann für die Zahnbogenkurve 500 des Oberkiefers und die Zahnbogenkurve des Unterkiefers und für die zusammengeführte patientenspezifische Zahnbogenkurve eine ROI ermittelt werden. Alternativ kann auf die ROI der Zahnbogenkurven für Ober- und Unterkiefer verzichtet werden und stattdessen nur für die zusammengeführte patientenspezifische Zahnbogenkurve die ROI ermittelt werden.

Die Auswerteeinheit 200 kann die ermittelte Zahnbogenkurve 500 basierend auf einer Lage und/oder Breite des Kiefers, insbesondere vorliegend beispielhaft basierend auf einer Lage und/oder Breite des Oberkiefers, anpassen. Hierfür ermittelt die Auswerteeinheit 200 die Lage und/oder die Breite des Kiefers, bezogen auf das beschriebene Beispiel die Lage und/oder Breite des Oberkiefers. Diese Ermittlung kann beispielsweise unter Berücksichtigung der ermittelten Aufbisspunkte 400 erfolgen. Beispielhaft bezugnehmend auf die ermittelte Breite des Kiefers, passt die Auswerteeinheit 200 die Zahnbogenkurve 500 unter Berücksichtigung der ermittelten Breite an. Bei einem sehr breiten Oberkiefer kann die Zahnbogenkurve 500 beispielsweise derart angepasst werden, dass sich beispielsweise eine breite Zahnbogenkurve 500a ergibt. Bei einem sehr schmalen Oberkiefer kann die Zahnbogenkurve 500 beispielsweise derart angepasst werden, dass sich beispielsweise eine schmale Zahnbogenkurve 500b ergibt. Beispielhaft wird vorliegend angenommen, dass die Zahnbogenkurve 500 nicht oder nur minimal angepasst / verändert wird, so dass sich nach Anpassung die gezeigte Zahnbogenkurve 500 ergibt. Die Anpassung der Zahnbogenkurve 500 kann auch als Kurvenfit bezeichnet werden.

Zusammengefasst kann somit die Zahnbogenkurve 500 unter Berücksichtigung von Lage und Breite des Kiefers, beispielsweise des Oberkiefers, angepasst werden. Die Anpassung kann durchgeführt werden auf die Zahnbogenkurve des Oberkiefers und/oder auf die Zahnbogenkurve des Unterkiefers und/oder auf die zusammengeführte patientenspezifische Zahnbogenkurve.

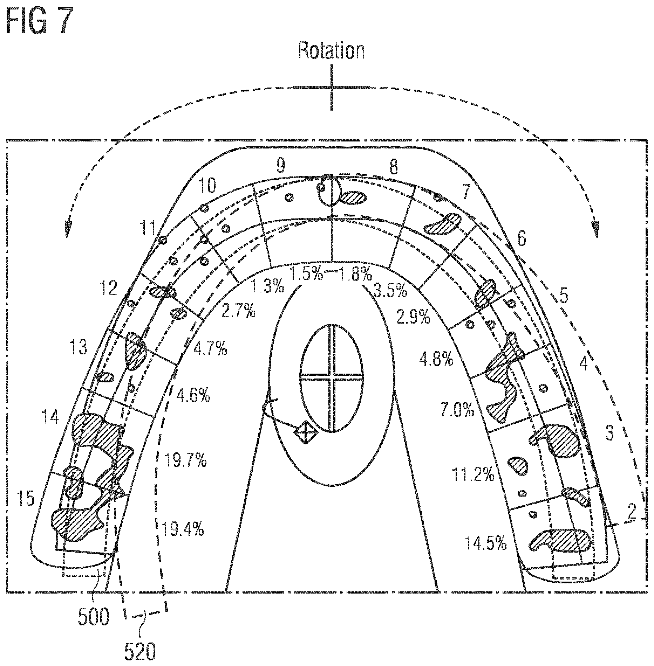

In Figur 7 wird schematisch dargestellt, wie die Auswerteeinheit 200 eine Rotation in der Horizontalebene mitberücksichtigen kann. Hierfür kann die Auswerteeinheit 200 eine Horizontalebene anhand der ermittelten Aufbisspunkte 400 ermitteln. Durch eine entsprechende Drehung in der Horizontalebene kann die Zahnbogenkurve 500 in die richtige Orientierung gebracht werden. Beispielsweise kann die Okklusalebene möglichst nahe an die Horizontalebene gebracht werden oder der Horizontalebene entsprechen. Beispielhaft ist in Figur 7 gezeigt, wie durch Rotation die Zahnbogenkurve 500 in eine gedrehte Zahnbogenkurve 520 überführt werden kann. Beispielsweise kann die parabelförmige Zahnbogenkurve 500, genauer gesagt die gesamte ROI um die Zahnbogenkurve 500, um einen Scheitelpunkt (zwischen Zähnen 11;21 und 31; 41) der Parabel in der Okklusalebene in die gedrehte Zahnbogenkurve 520 samt deren ROI gedreht werden.

Durch Kurvenfit kann daher auch eine Rotation in der Horizontalebene gemessen und/oder berücksichtigt werden. Dadurch kann die Zahnbogenkurve 500 automatisch angepasst werden.

Durch eine entsprechend große Drucksensorfläche kann ebenfalls die Sagittalebene des Patienten bestimmt werden. Bei bekannter tatsächlicher Sagittalebene kann eine Rotation der Zahnbogenkurve 500 berechnet werden. Beispielsweise kann, wie in Figur 7 gezeigt, die Zahnbogenkurve 500 in die gedrehte Zahnbogenkurve 520 überführt werden.

Eine Gerätebewegungskurve, beispielsweise eine Panoramaaufnahmekurve eines Röntgengeräts, kann basierend auf der ermittelten patientenspezifischen Zahnbogenkurve 500 ermittelt werden. Beispielsweise kann ein Rotationsstartpunkt angepasst werden. Ferner kann über die Anpassung der Gerätebewegungskurve ein Verhältnis eines Abstands zwischen Röntgenstrahler und Patient / Objekt zu einem Abstand zwischen Patient / Objekt und Röntgendetektor angepasst werden.

Durch eine entsprechend große Drucksensorfläche kann ebenfalls die Sagittalebene des Patienten bestimmt werden. Bei bekannter tatsächlicher Sagittalebene kann die Gerätebewegungskurve ebenfalls angepasst werden.

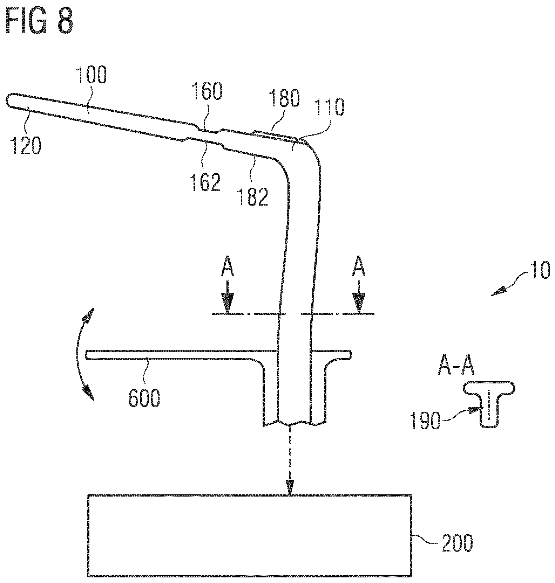

In Figur 8 ist eine Variante der Vorrichtung 10 aus Figur 1 in einer Seitenansicht dargestellt.

Die Aufbissanordnung 100 gemäß dieser Variante weist eine obere Einkerbung 160 (siehe auch Figur 2) für die vorderen Schneidezähne (Zähne Nr. 11, 21) des Oberkiefers sowie eine untere Einkerbung 162 (siehe auch Figur 2) für die vorderen Schneidezähne (Zähne Nr. 31, 41) des Unterkiefers auf. Die Schneidezähne des Oberkiefers können in die Einkerbung 160 und die Schneidezähne des Unterkiefers können in die Einkerbung 162 eingreifen. An dem Schaft 110 der Aufbissanordnung 100 sind ferner Dehnungsmesstreifen (DMS) 180, 182 angeordnet. Genauer gesagt sind an einer Oberseite des Schafts 110 ein oberer DMS 180 und an der Unterseite des Schafts 110 ein unterer DMS 182 angeordnet oder aufgebracht. Mittels der DMS 180, 182 lässt sich eine Krafteinwirkung des Oberkiefers auf den DMS 180 und eine Krafteinwirkung des Unterkiefers auf den DMS 182 bestimmen. Ist die Krafteinwirkung gleich groß, kann eine Neigung einer Okklusalebene / Okklusionsebene unverändert in neutraler Position verbleiben. Ist die Krafteinwirkung des Oberkiefers größer als die des Unterkiefers, kann entsprechend die Neigung der Okklusalebene von der Auswerteeinheit 200 angepasst werden, beispielsweise entsprechend dem Unterschiedsbetrag anders geneigt werden.

Das heißt, die Neigung der Okklusalebene kann durch die auftretende Krafteinwirkung im Schaftbereich 110 der Aufbissschiene 120 bestimmt werden. Wenn die DMS 180, 182 an der Ober- und Unterseite des Schaftes 110 gleich große Werte liefern, wird keine Kraft auf den Schaft 110 durch den Patienten ausgeübt. Da die Aufbissschiene 120 im korrekten Winkel für die Bildstellung angeordnet ist, ist somit auch die Okklusalebene in der richtigen Lage. Folglich kann die Okklusalebene basierend auf der ermittelten Neigung geneigt werden. Es können somit Rotationen auf der Okklusalebene detektiert werden und die Position des Patienten korrigiert (=Feedbacksystem) und/oder die Fehlpositionierung mit in die Bahnkurve, beispielsweise die Panoramaaufnahmekurve, mit eingerechnet werden. Wie in Figur 8 gezeigt, verläuft die Aufbissanordnung 100, alternativ die Aufbissschiene 110 der Aufbissanordnung 100, nicht parallel zu einer Horizontalen, sondern geneigt zu einer Horizontalen. Ferner verläuft die Aufbissanordnung 100, alternativ die Aufbissschiene 110 der Aufbissanordnung 100, normalerweise schräg oder geneigt zu einer Okklusalebene (Okklusionsebene). Zudem ist die Aufbissanordnung 100 oder der Schaft 110 der Aufbissanordnung 100 durch die Einkerbungen 160, 162 sowie die DMS 180, 182 in der in Figur 8 gezeigten Seitenansicht leicht wellenförmig ausgebildet.

Wie oben skizziert, kann die Neigung der Okklusalebene angepasst werden. Es können somit Rotationen auf der Okklusalebene detektiert werden und die Position des Patienten korrigiert (=Feedbacksystem) und/oder die Fehlpositionierung mit in die Bahnkurve, beispielsweise die Panoramaaufnahmekurve, mit eingerechnet werden. Beispielsweise ist es bei einem Panoramabild empfehlenswert, dass die Patientenwirbelsäule leicht überstreckt ist, um den Frontzahnbereich möglichst artefaktfrei darstellen zu können. Des Weiteren wird für gewöhnlich bei der Aufnahme des Frontzahnbereiches die applizierte Röntgendosis erhöht, da hier die Strahlen die weiteste Distanz durch das Gewebe zurücklegen müssen und somit am meisten Artefaktinformationen aufsammeln. Der Patientenkopf sollte daher idealerweise leicht nach unten geneigt sein. Dies kann indirekt dadurch erreicht werden, indem die Frankfurter Horizontale und die Gerätehorizontale deckungsgleich sind. Der Winkel zwischen Frankfurter Horizontale und Okklusalebene ist bekannt und beträgt ca. 5,6°. Somit kann per Messung die korrekte Lage der Ebenen sichergestellt bzw. hergestellt werden. Dadurch kann von einer korrekten Überstreckung der Wirbelsäule ausgegangen werden und die Verwendung eines Frankfurter Lasers im Gerät kann entfallen. Optional kann auch die Röntgendosis gesenkt werden, da ein Durchstrahlen einer nichtüberstreckten Wirbelsäule ausgeschlossen werden kann.

Generell ist es bei der Aufnahme eines Zahns oder mehrerer Zähne möglich, einen sogenannten Field of View (FOV), d. h. ein Sichtfeld oder einen Sichtbereich, eines bildgebenden Systems zu verringern, beispielsweise zu minimieren. Auf diese Weise kann eine Strahlendosis verringert, beispielsweise minimiert, werden. Zudem kann eine verbesserte, beispielsweise eine bestmögliche, Bildqualität erreicht werden. Zum Beispiel ist es möglich, das Sichtfeld auf einen einzigen Zahn zu richten. Dieser Zahn kann beispielsweise von der Spitze bis zur Wurzel aufgenommen werden.

Zusätzlich kann eine Neigung der Kinnstütze 600 erfasst und gemessen werden. Die ermittelte Neigung der Kinnstütze kann von der Auswerteeinheit 600 in den Auswertungen berücksichtigt werden. Auf diese Weise kann eine Stauchung und/oder Dehnung des Schaftes 110 der Aufbissanordnung 100 erfasst werden.

In Figur 8 ist zudem ein Schnitt A-A eingezeichnet. In der zugehörigen Schnittfigur ist eine Leitung 190, beispielsweise eine Kupfer-Leitung, zu erkennen. Über die Leitung 190 können die erfassten Druckinformationen / Drucksignale der Drucksensoren 140 140 zu der Auswerteeinheit 200 geleitet werden oder zu einer Einheit, von der die Druckinformationen der Drucksensoren 140 drahtlos oder drahtgebunden an die Auswerteeinheit 200 übermittelt werden können.

Durch die beschriebene Vorrichtung und das beschrieben Verfahren kann eine Kiefergeometrie zumindest nahezu exakt ermittelt werden.

Die Gesamtheit der Messdaten in Verbindung mit den Bewegungsmöglichkeiten des bildgebenden Systems ermöglicht eine Korrektur von Positionierungsfehlern, die durch den Anwender oder den Patienten entstehen können.

Ferner werden folgende Vorteile erreicht. Durch Messung des Kieferbogens kann eine automatische Auswahl der Kieferbreite und/oder eine Möglichkeit der Anpassung einer patientenindividuellen / patientenspezifischen Bahnkurve, insbesondere Panoramakurve, erreicht werden. Es kommt zu keiner oder weniger Interaktion bei der Positionierung und folglich zu weniger Fehlerpotential. Es findet ein Ausgleich möglicher Positionierungsfehler statt und somit kommt es zu einer Anpassung in der Bewegung eines C-Bogens (Startpunkt Translation und Rotation). Durch Integration eines Feedbacksystems kann eine bessere Positionierung erreicht werden. Es kann eine Speicherung der Messdaten zur Auswertung der Kiefergeometrie erfolgen. Es kommt zu einer besseren Positionierung für ein Digitales Volumentomographie (DVT) Volumen.

Insgesamt werden die Bildqualität und die Bedienerfreundlichkeit gesteigert.

Im Gegensatz zum Stand der Technik ist weder eine Vorberechnung noch eine Nachberechnung des Zahnbogens nötig, sondern eine direkt vom Patienten abnehmbare Zahnbogenkurve. Eine aufwändige vorgelagerte Erstellung / Festlegung (in einem extra Arbeitsschritt) der Kiefergeometrie oder eine Berücksichtigung von vorhandenen, teilweise ggf. falschem oder schlechten Bildmaterial kann somit ersatzlos entfallen.

Das heißt, es können Nachberechnungen, Nachbearbeitungen, Positionierungslaser, Falschpositionierungen durch Personal und/oder zusätzliche Aufnahmen (Scout) entfallen.

## Patentansprüche

1. Vorrichtung (10) zur Ermittlung von Informationen über zumindest einen Abschnitt eines Zahnbogens, aufweisend:
eine Aufbissanordnung (100) mit mindestens einem Drucksensor, (140) wobei die Aufbissanordnung ausgebildet ist, zwischen zumindest einem Abschnitt einer Zahnreihe (300) eines Oberkiefers und zumindest einem Abschnitt einer Zahnreihe eines Unterkiefers aufgenommen zu werden, und wobei der mindestens eine Drucksensor ausgebildet ist, Druckinformationen über von zumindest dem Abschnitt der Zahnreihe des Oberkiefers und/oder von zumindest dem Abschnitt der Zahnreihe des Unterkiefers auf den mindestens einen Drucksensor ausgeübten Druck zu erfassen; und
eine Auswerteeinheit (200), die ausgebildet ist, basierend auf den von dem mindestens einen Drucksensor erfassten Druckinformationen, Informationen über zumindest einen Abschnitt eines Zahnbogens zu ermitteln,
wobei die Aufbissanordnung ferner mindestens eine Messeinrichtung mit mindestens einen Dehnungsmessstreifen an einer Oberseite eines Schaftes (110) der Aufbissanordnung und/oder mindestens einen Dehnungsmessstreifen an einer Unterseite des Schaftes der Aufbissanordnung aufweist.

2. Vorrichtung nach Anspruch 1, wobei die Auswerteeinheit ausgebildet ist, als die Informationen über zumindest den Abschnitt des Zahnbogens, Informationen über zumindest einen Abschnitt eines Zahnbogens des Oberkiefers und/oder Informationen über zumindest einen Abschnitt eines Zahnbogens des Unterkiefers zu ermitteln.

3. Vorrichtung nach Anspruch 1, wobei die Auswerteeinheit ausgebildet ist, als die Informationen über zumindest den Abschnitt des Zahnbogens, Informationen über zumindest einen Abschnitt eines, aus Informationen über zumindest einen Abschnitt eines Zahnbogens des Oberkiefers und Informationen über zumindest einen Abschnitt eines Zahnbogen des Unterkiefers, gemittelten Zahnbogens zu ermitteln.

4. Vorrichtung nach einem der Ansprüche 1 bis 3, wobei die Auswerteeinheit ausgebildet ist, basierend auf den von dem mindestens einen Drucksensor erfassten Druckinformationen, Informationen über eine Breite und/oder einer Lage eines den Oberkiefer und den Unterkiefer aufweisenden Kiefers zu ermitteln.

5. Vorrichtung nach einem der Ansprüche 1 bis 4, wobei der mindestens eine Drucksensor ausgebildet und angeordnet ist, als die Druckinformationen, Informationen über einen oder mehrere Aufbisspunkte in der Zahnreihe des Oberkiefers und/oder in der Zahnreihe des Unterkiefers zu erfassen.

6. Vorrichtung nach einem der Ansprüche 1 bis 5, wobei der mindestens eine Drucksensor ausgebildet und angeordnet ist, als die Druckinformationen, Informationen über bukkale und/oder palatinale Positionen von einem oder mehreren Zahnhöckern und/oder von einem oder mehreren Schneideflächen von Zähnen der Zahnreihe des Oberkiefers und/oder von Zähnen der Zahnreihe des Unterkiefers zu erfassen.

7. Vorrichtung nach einem der Ansprüche 1 bis 6, wobei der mindestens eine Drucksensor ein Sensor-Array mit Einzelsensoren und/oder Dünnfilmsensoren und/oder Foliensensoren aufweist, oder als Sensor-Array ausgebildet ist, welches Einzelsensoren und/oder Dünnfilmsensoren und/oder Foliensensoren aufweist.

8. Vorrichtung nach einem der Ansprüche 1 bis 7, wobei die Auswerteeinheit ausgebildet ist, basierend auf den von dem mindestens einen Drucksensor erfassten Druckinformationen, Informationen über eine Sagittalebene zu ermitteln.

9. Vorrichtung nach einem der Ansprüche 1 bis 8, wobei die Auswerteeinheit ausgebildet ist, basierend auf von der mindestens einen Messeinrichtung erfassten Informationen, Informationen über eine Neigung einer Okklusalebene zu ermitteln.

10. Verfahren zur Ermittlung von Informationen über zumindest einen Abschnitt eines Zahnbogens, umfassend:
Bereitstellen einer Aufbissanordnung (100) mit mindestens einem Drucksensor (140) und mindestens einer Messeinrichtung mit mindestens einem Dehnungsmessstreifen an einer Oberseite eines Schaftes (110) der Aufbissanordnung und/oder mindestens einem Dehnungsmessstreifen an einer Unterseite des Schaftes der Aufbissanordnung;
Aufnehmen der Aufbissanordnung zwischen zumindest einem Abschnitt einer Zahnreihe (300)
eines Oberkiefers und/oder zumindest einem Abschnitt einer Zahnreihe eines Unterkiefers;
Erfassen von Druckinformationen über von zumindest dem Abschnitt der Zahnreihe des Oberkiefers und/oder von zumindest dem Abschnitt der Zahnreihe des Unterkiefers auf den mindestens einen Drucksensor der Aufbissanordnung ausgeübten Druck; und
Ermitteln, durch eine Auswerteeinheit (200), basierend auf den von dem mindestens einen Drucksensor erfassten Druckinformationen, von Informationen über zumindest einen Abschnitt eines Zahnbogens.

## Claims

1. A device (10) for determining information about at least a portion of a dental arch, comprising:
a bite block assembly (100) with at least one pressure sensor (140), wherein the bite block assembly is configured to be received between at least a portion of a row of teeth of an upper jaw and at least a portion of a row of teeth of a lower jaw, and wherein the at least one pressure sensor is configured to sense pressure information about pressure exerted by at least the portion of the row of teeth of the upper jaw and/or by at least the portion of the row of teeth of the lower jaw on the at least one pressure sensor; and
an evaluation unit (200) which is configured to determine information about at least a portion of a dental arch based on the pressure information sensed by the at least one pressure sensor,
wherein the bite block assembly further comprises at least one measuring system with at least one strain gauge on an upper side of a shaft (110) of the bite block assembly and/or at least one strain gauge on an underside of the shaft of the bite block assembly.

2. The device according to claim 1, wherein the evaluation unit is configured to determine, as the information about at least the portion of the dental arch, information about at least a portion of a dental arch of the upper jaw and/or information about at least a portion of a dental arch of the lower jaw.

3. The device according to claim 1, wherein the evaluation unit is configured to determine, as the information about at least the portion of the dental arch, information about at least a portion of a dental arch averaged from information about at least a portion of a dental arch of the upper jaw and information about at least a portion of a dental arch of the lower jaw.

4. The device according to any one of claims 1 to 3, wherein the evaluation unit is configured to determine information about a width and/or a position of a jaw including the upper jaw and the lower jaw, based on the pressure information sensed by the at least one pressure sensor.

5. The device according to any one of claims 1 to 4, wherein the at least one pressure sensor is configured and arranged to sense, as the pressure information, information about one or more bite points in the row of teeth of the upper jaw and/or in the row of teeth of the lower jaw.

6. The device according to any one of claims 1 to 5, wherein the at least one pressure sensor is configured and arranged to sense, as the pressure information, information about buccal and/or palatal positions of one or more tooth cusps and/or of one or more cutting surfaces of teeth of the row of teeth of the upper jaw and/or of teeth of the row of teeth of the lower jaw.

7. The device according to any one of claims 1 to 6, wherein the at least one pressure sensor includes a sensor array with individual sensors and/or thin-film sensors and/or foil sensors or is configured as a sensor array which includes individual sensors and/or thin-film sensors and/or foil sensors.

8. The device according to any one of claims 1 to 7, wherein the evaluation unit is configured to determine information about a sagittal plane, based on the pressure information sensed by the at least one pressure sensor.

9. The device according to any one of claims 8, wherein the evaluation unit is configured to determine information about an inclination of an occlusal plane, based on information sensed by the at least one measuring system.

10. A method for determining information about at least a portion of a dental arch, comprising:
providing a bite block assembly (100) with at least one pressure sensor (140) and at least one measuring system with at least one strain gauge on an upper side of a shaft (110) of the bite block assembly and/or at least one strain gauge on an underside of the shaft of the bite block assembly;
receiving the bite assembly between at least a portion of a row of teeth (300) of an upper jaw and/or at least a portion of a row of teeth of a lower jaw;
sensing pressure information about pressure exerted by at least the portion of the row of teeth of the upper jaw and/or by at least the portion of the row of teeth of the lower jaw on the at least one pressure sensor of the bite block assembly; and
determining, by an evaluation unit (200), information about at least a portion of a dental arch, based on the pressure information sensed by the at least one pressure sensor.

## Revendications

1. Dispositif (10) pour déterminer des informations sur au moins une partie d'une arcade dentaire, comprenant :
un agencement d'occlusion (100) comportant au moins un capteur de pression (140), l'agencement d'occlusion étant conçu pour être placé entre au moins une partie d'une rangée de dents (300) d'une mâchoire supérieure et au moins une partie d'une rangée de dents d'une mâchoire inférieure, et dans lequel ledit au moins un capteur de pression est conçu pour détecter des informations de pression sur la pression exercée sur ledit au moins un capteur de pression par au moins la partie de la rangée de dents de la mâchoire supérieure et/ou par au moins la partie de la rangée de dents de la mâchoire inférieure ; et
une unité d'évaluation (200) conçue pour déterminer des informations sur au moins une partie d'une arcade dentaire sur la base des informations de pression détectées par ledit au moins un capteur de pression,
l'agencement d'occlusion comprenant en outre au moins un moyen de mesure comportant au moins une jauge de contrainte sur une face supérieure d'une tige (110) de l'agencement d'occlusion et/ou au moins une jauge de contrainte sur une face inférieure de la tige de l'agencement d'occlusion.

2. Dispositif selon la revendication 1, dans lequel l'unité d'évaluation est conçue pour déterminer les informations sur au moins la partie de l'arcade dentaire, des informations sur au moins une partie d'une arcade dentaire de la mâchoire supérieure et/ou des informations sur au moins une partie d'une arcade dentaire de la mâchoire inférieure.

3. Dispositif selon la revendication 1, dans lequel l'unité d'évaluation est conçue pour déterminer les informations sur au moins la partie de l'arcade dentaire, des informations sur au moins une partie d'une arcade dentaire moyenne, calculée à partir d'informations sur au moins une partie d'une arcade dentaire de la mâchoire supérieure et d'informations sur au moins une partie d'une arcade dentaire de la mâchoire inférieure.

4. Dispositif selon l'une des revendications 1 à 3, dans lequel l'unité d'évaluation est conçue pour déterminer des informations sur une largeur et/ou une position d'une mâchoire comprenant la mâchoire supérieure et la mâchoire inférieure sur la base des informations de pression détectées par ledit au moins un capteur de pression.

5. Dispositif selon l'une des revendications 1 à 4, dans lequel ledit au moins un capteur de pression est conçu et disposé de manière à détecter, en tant qu'informations de pression, des informations sur un ou plusieurs points d'occlusion dans la rangée de dents de la mâchoire supérieure et/ou dans la rangée de dents de la mâchoire inférieure.

6. Dispositif selon l'une des revendications 1 à 5, dans lequel ledit au moins un capteur de pression est conçu et disposé de manière à détecter, en tant qu'informations de pression, des informations sur les positions buccales et/ou palatines d'une ou plusieurs cuspides dentaires et/ou d'une ou plusieurs surfaces de coupe de dents de la rangée de dents de la mâchoire supérieure et/ou de dents de la rangée de dents de la mâchoire inférieure.

7. Dispositif selon l'une des revendications 1 à 6, dans lequel ledit au moins un capteur de pression comprend un réseau de capteurs comportant des capteurs individuels et/ou des capteurs à couche mince et/ou des capteurs à film sensible, ou est réalisé sous la forme d'un réseau de capteurs qui comprend des capteurs individuels et/ou des capteurs à couche mince et/ou des capteurs à film sensible.

8. Dispositif selon l'une des revendications 1 à 7, dans lequel l'unité d'évaluation est conçue pour déterminer des informations sur un plan sagittal sur la base des informations de pression détectées par ledit au moins un capteur de pression.

9. Dispositif selon l'une des revendications 1 à 8, dans lequel l'unité d'évaluation est conçue pour déterminer des informations sur l'inclinaison d'un plan occlusal sur la base des informations détectées par ledit au moins un moyen de mesure.

10. Procédé pour déterminer des informations sur au moins une partie d'une arcade dentaire, comportant :
la mise à disposition d'un agencement d'occlusion (100) comportant au moins un capteur de pression (140) et au moins un moyen de mesure comportant au moins une jauge de contrainte sur une face supérieure d'une tige (110) de l'agencement d'occlusion et/ou au moins une jauge de contrainte sur une face inférieure de la tige de l'agencement d'occlusion ;
le placement de l'agencement d'occlusion entre au moins une partie d'une rangée de dents (300) d'une mâchoire supérieure et/ou au moins une partie d'une rangée de dents d'une mâchoire inférieure ;
la détection d'informations de pression sur la pression exercée par au moins la partie de la rangée de dents de la mâchoire supérieure et/ou par au moins la partie de la rangée de dents de la mâchoire inférieure sur ledit au moins un capteur de pression de l'agencement d'occlusion ; et
la détermination, par une unité d'évaluation (200), sur la base des informations de pression détectées par ledit au moins un capteur de pression, d'informations sur au moins une partie d'une arcade dentaire.
